# EUROPÄISCHE PATENTSCHRIFT

(11) **EP 4 308 078 B1**
(45) Veröffentlichungstag und Bekanntmachung des Hinweises auf die Patenterteilung: **12.03.2025**
(21) Anmeldenummer: 22716378.9
(22) Anmeldetag: 17.03.2022
(51) Int. Cl.: A61K 9/00, A61K 9/70, A61K 31/00

(54) **GEROLLTE ORAL THIN FILMS MIT HOHER WIRKSTOFFBELADUNG**
ROLLED ORAL THIN FILMS HAVING A HIGH LEVEL OF ACTIVE-INGREDIENT LOADING
FILM EXTRA-FIN ENROULÉ À USAGE ORAL, À CHARGE ÉLEVÉE EN PRINCIPE ACTIF

(30) Priorität: 17.03.2021 DE 102021106491
(43) Veröffentlichungstag der Anmeldung: 24.01.2024
(73) Patentinhaber: LTS Lohmann Therapie-Systeme AG, 56626 Andernach (DE)
(72) Erfinder: MÜLLER, Markus, 53840 Troisdorf (DE); SCHMITZ, Christoph, 56598 Rheinbrohl (DE); LINN, Michael, 55596 Waldböckelheim (DE)
(74) Vertreter: Held, Stephan
(86) Internationale Anmeldenummer: PCT/EP2022/057068
(87) Internationale Veröffentlichungsnummer: WO 2022/195044

(56) Entgegenhaltungen:
- WO-A1-2018/224591
- DE-A1- 19 946 822
- DE-C1- 19 715 794
- US-A- 3 625 214

## Beschreibung

Die vorliegende Erfindung betrifft ein Verfahren zur Herstellung von aufgerollten Oral-Thin-Film-Laminaten, sowie nach dem Verfahren hergestellten aufgerollten Laminaten.

### Stand der Technik

Orale Dünnfilme (Oral Thin Films), auch transmukosale Verabreichungssysteme genannt, sind dünne wirkstoffhaltige Filme auf Polymerbasis, die, wenn sie auf eine Schleimhaut, insbesondere die Mundschleimhaut, aufgebracht werden, den Wirkstoff direkt in diese abgeben. Diese Darreichungssysteme haben den Vorteil, dass der Wirkstoff größtenteils durch die Schleimhaut resorbiert wird und damit der "First-Pass Metabolismus", der bei der konventionellen Darreichungsform eines Wirkstoffs in Tablettenform beachtet werden muss, vermieden wird.

Eine Schwierigkeit bei der Herstellung von oralen Dünnfilmen stellen in der Praxis Filme mit hohen Flächengewichten dar. Um hohe Flächengewichte zu realisieren ist es erforderlich, entweder den zur Trocknung vorgesehenen Film als vergleichsweise dicken Film herzustellen, was zu Problemen beim Trocknungsprozess führt. Alternativ können Filme mit hohen Flächengewichten auch durch schichtweises Applizieren von mehreren Schichten aufeinander mit zwischengeschalteten Trocknungsschritten realisiert werden, was jedoch nicht in allen Fällen zu einem Filmprodukt mit zufriedenstellender struktureller Integrität führt. Zudem sind bei einem schichtweisen Aufbau von oralen Dünnfilmen mehrfache Aufbring- und Trocknungsschritte erforderlich, was deren Herstellung relativ kompliziert macht.

Sollen mit einem oralen Dünnfilm größere Mengen an Wirkstoff verabreicht werden, so stellt sich zudem das Problem, dass am Applikationsort, zum Beispiel im Gaumen oder in der Wange, nur begrenzt Platz zur Verfügung steht, so dass die Größe des Filmprodukts durch diese Rahmenbedingungen begrenzt ist.

WO 2018/224591 A1 offenbart flächenförmige, in wässriger Umgebung sich auflösende oder zersetzende Darreichungsformen zur Wirkstofffreisetzung in einer Körperöffnung, umfassend eine Polymermatrix in Form eines Hohlräume aufweisenden verfestigten Schaums.

DE 199 46 822 A1 offenbart eine Wirk- und/oder Hilfsstoffe enthaltende Zubereitung für die zeitlich und/oder hinsichtlich der Dosis steuerbare Freisetzung dieser Stoffe, die mindestens zwei Schichten in gerollter oder gefalteter Form enthält. Dabei ist die erste dieser Schichten, die den mindestens einen Wirk- oder Hilfsstoff enthält, so konstruiert, dass mindestens einer der Parameter Dicke der Schicht, Breite der Schicht und Konzentration des Wirkstoffs in dieser Schicht nicht konstant ist.

Vor diesem Hintergrund besteht ein Bedarf nach Verabreichungsformen für pharmazeutische Wirkstoffe und andere Stoffe, die in größerer Menge verabreicht werden. Die Verabreichungsformen sollten die Vorteile eines Applikationssystems auf Basis einer sich schnell auflösenden Dosierungsform wie einem oralen Dünnfilm realisieren, und zusätzlich bei relativ kleinen äußeren Dimensionen eine möglichst hohe Wirkstoffmenge transportieren. Bei der Herstellung der Verabreichungsformen sollten die bekannten Probleme von oralen Dünnfilmen mit hohem Flächengewicht vermieden werden.

Die vorliegende Erfindung befasst sich mit diesem Bedarf.

### Beschreibung der Erfindung

Es wurde gefunden, dass gerollte Laminate von Filmen, die auf den gleichen Materialien beruhen wie konventionelle orale Dünnfilme, in einem begrenzten Volumen einen hohen Wirkstoffgehalt transportieren können und mit einem hohen Gesamtgewicht formuliert werden können. Solche gerollten Laminate können leicht in die Wangentasche oder neben bzw. unter die Zunge gelegt werden, und dort den Wirkstoff freisetzen.

Demzufolge betrifft ein erster Aspekt der vorliegenden Erfindung ein Verfahren zur Herstellung einer wasserlöslichen oder wasserdispergierbaren Wirkstoff-Verabreichungsform, dass die Schritte
(a) Herstellen eines als orale Filmverabreichungsform geeigneten Films mit einem Gehalt an Wirkstoff, wobei die Filmverabreichung als verfestigter Schaum vorliegt,
(b) Aktivieren einer Oberfläche des in (a) hergestellten Films zur Generierung einer klebrigen Oberfläche und
(c) Aufrollen des in (b) aktivierten Films unter Bildung eines aufgerollten Laminats umfasst.

Im Rahmen dieses Verfahrens ist es zweckmäßig, wenn die in (b) generierte klebrige Oberfläche im Schritt (c) so positioniert ist, dass sich die klebrige Seite auf der Innenseite befindet, so dass in dem fertigen aufgerollten Laminat keine klebrigen Reste an der Außenseite verbleiben.

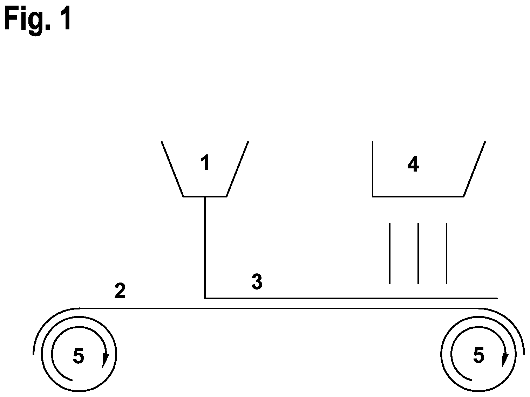

Das Herstellen des Films in (a) erfolgt üblicherweise durch Aufbringen einer fließfähigen Masse der Zusammensetzung, die im weiteren den Film bildet, und Trocknen der Masse unter Bildung des Films. Eine geeignete Vorrichtung zur Herstellung entsprechender Filme ist schematisch in Fig. 1 dargestellt. Die fließfähigen Masse wird über eine geeignete Einheit **1** auf ein Substratband **2** aufgebracht, wobei sich auf dem Substratband ein Film **3** ausbildet. Durch Bewegung, z.B. von entsprechenden Antriebsrollen **5,** wird der Film anschließend in einen Trockner **4** geführt und dort zu einem verfestigten Film getrocknet. Nach dem Trocknen kann der Film von Substratband **2** getrennt werden.

In einer bevorzugten Ausführungsform des erfindungsgemäßen Verfahrens erfolgt das Aktivieren der Oberfläche und das Generieren einer klebrigen Oberfläche in (b) durch Anlösen der Oberfläche. Für ein Anlösen kann z.B. eine geringe Menge eines geeigneten Lösungsmittels, wie Wasser oder ein pharmazeutisch akzeptabler Alkohol, z.B. Ethanol, auf die Oberfläche des Films dampfförmig einwirken bzw. in flüssiger Form aufgebracht werden, wodurch der Film im oberflächennahen Bereich in begrenztem Ausmaß quillt bzw. angelöst wird. Mit dem so begrenzt gequollenem bzw. angelösten Film kann anschließend durch Aufrollen ein Verbund zwischen der aktivierten (ersten) Oberfläche und der nicht aktivierten (zweiten) Oberfläche des aufgerollten Films realisiert werden. Der Vorteil des Quellens gegenüber dem Anlösen liegt darin, dass der Film bei diesem Schritt kohärent bleibt. Nach dem Aufrollen kann sich die geringe Menge des Wassers bzw. des pharmazeutisch akzeptablen Alkohols, welche zum Quellen/Anlösen der Oberfläche des Films gedient hat, durch Diffusion in dem aufgerollten Film verteilen und/oder durch Trocknung daraus entfernt werden. Weil im Fall dieser Art der Aktivierung keine Zwischenschicht entsteht, besteht das aufgerollte Laminat ausschließlich aus dem Material, das den Film bildet.

In einer weiteren bevorzugten Ausführungsform des erfindungsgemäßen Verfahrens erfolgt das Aktivieren der Oberfläche und das Generieren einer klebrigen Oberfläche in (b) durch Anschmelzen der Oberfläche, beispielsweise in dem über eine geeignete Heizvorrichtung die Oberfläche erwärmt und partiell verflüssigt wird.

In einer alternativen bevorzugten Ausführungsform erfolgt das Aktivieren der Oberfläche und das Generieren einer klebrigen Oberfläche in (b) durch das Aufbringen einer selbstklebenden Schicht, wobei die selbstklebende Schicht aus einem wasserlöslichen oder wasserdispergierbaren Material gebildet wird. Bevorzugt für die selbstklebende Schicht ist ein wasserlösliches Material.

Eine solche selbstklebende Schicht wird z.B. hergestellt, indem ein wasserlösliches und/oder wasserdispergierbares Material in Wasser und/oder einem pharmazeutisch akzeptablen Lösungsmittel, vorzugsweise einem Alkohol wie z.B. Ethanol, gelöst wird und in einer dünnen Schicht auf eine (dehäsiv ausgerüstete) Trägerfolie ausgestrichen wird. Diese Schicht wird bis zu einem Restwassergehalt bzw. einem Restlösemittelgehalt getrocknet, der dieser Schicht noch eine gewisse Klebrigkeit verleiht. In diesem Zustand wird diese Schicht nun auf die (erste) Oberfläche des in Schritt (a) hergestellten Films aufgetragen, wobei die Trägerfolie abgezogen werden kann. Dies ermöglicht, dass die "selbstklebende Schicht" eine sehr geringe Schichtdicke von 1 bis 100 µm, insbesondere 2 bis 20 und besonders bevorzugt 4 bis 10 µm aufweisen kann. Auch hier kann sich der Restwasser- bzw. Restlösemittelgehalt im aufgerollten Laminat durch Diffusion verteilen bzw. durch Trocknung verringert werden.

Diese Art der Aktivierung der (ersten) Oberfläche des Films und Ausrüsten mit einer klebrigen Oberfläche ist insbesondere bei Verwendung eines schaumförmigen Films in Schritt (a) von Vorteil. Das Risiko eines Quellens bzw. Anlösens des Films wird weitgehend ausgeschlossen, so dass die Schaumstruktur des Films nicht beeinträchtigt wird. Das (getrocknete) Material der Klebeschicht verbleibt im aufgerollten Laminat zwischen der "aktivierten" und der "nichtaktivierten" Oberfläche des Films.

Das Aufbringen einer selbstklebenden Schicht kann durch leichtes Andrücken der selbstklebenden Schicht auf die Wirkstoff-haltige Schicht unterstützt werden, beispielsweise mit Hilfe Gummirolle. Bei dem Aufbringen von selbstklebenden Schichten ist dabei die Andrückkraft geeignet einzustellen, damit z.B. ein Wirkstoff-haltiger Schaumfilm durch das Andrücken nicht beschädigt wird. Der Fachmann kann eine sinnvolle Andrückkraft hierbei fachmännisch durch geeignete Versuche ermitteln.

Eine dritte Art der Aktivierung der (ersten) Oberfläche des Films besteht in seinem Anschmelzen. Hierfür wird auf diese Oberfläche Energie (z.B. in Form von Wärme) aufgetragen, so dass - bei Verwendung eines schaumförmigen Films - eine Erweichung dieser Oberfläche erfolgt, die nach Aufrollen und danach stattfindendem Abkühlen des aufgerollten Laminats zu einer festen Verbindung der so aktivierten (ersten) Oberfläche mit der nicht aktivierten (zweiten) Oberfläche führt.

Gemäß dem im Vorstehenden beschriebenen Verfahren hergestellte aufgerollte Laminate können anschließend gegebenenfalls auf eine geeignete Länge geschnitten in üblicher Weise verpackt werden, z.B. mittels einer Schlauchbeutelmaschine.

In Bezug auf den als orale Filmverabreichungsform geeigneten Film unterliegt die vorliegende Erfindung keinen relevanten Beschränkungen mit der Maßgabe, dass der Film grundsätzlich als orale Filmverabreichungsform geeignet ist, d.h., dass er sich bei Kontakt mit Speichel relativ schnell löst und zerfällt, so dass der im Film enthaltene Hilfs- oder Wirkstoff freigesetzt wird. Die im Film enthaltenen Bestandteile sollten zudem pharmazeutisch akzeptable sein. Als geeignete filmbildende Materialien sind unter anderem wasserlösliche Polymere zu nennen, die pharmazeutisch akzeptabel sind. Besonders geeignete wasserlösliche Polymere sind beispielsweise Stärke und Stärkederivate, Dextrane; Cellulosederivate, wie Carboxymethylcellulose, Hydroxypropylcellulose, Hydroxyethylcellulose, Hydroxypropylmethylcellulose, Hydroxypropylethylcellulose, Natriumcarboxymethylcellulose, Ethyl- oder Propylcellulose; Polyacrylsäure, Polyacrylate, Polyvinylpyrrolidone, Polyvinylalkohol, Polyethylenoxidpoylmere, Polyacrylamide, Polyethylenglycol, Gelatine, Collagen, Alginate, Pektine, Pullulan, Traganth, Chitosan, Alginsäure, Arabinogalaktan, Galaktomannan, Agar, Agarose, Carrageen und natürliche Gumme. Besonders bevorzugt im Kontext der vorliegenden Erfindung sind wasserlösliche Polymere, die aus der Gruppe, umfassend Polyvinylalkohol, Polyethylenglycol, Polyethylenoxid, Cellulosederivate, Pullulan, Gelatine und Agar, ausgewählt sind. Meist bevorzugt ist im Kontext der vorliegenden Erfindung Polyvinylalkohol als wasserlösliches Polymer.

Der Anteil des filmbildenden Polymers im Film, der als orale Filmverabreichungsform geeignet ist, beträgt vorzugsweise 25 bis 85 Gew.-%, bevorzugt 50 bis 85 Gew.-% und weiter bevorzugt 60 bis 80 Gew.-%. In einigen Fällen kann, soweit dies die Integrität des Films nicht beeinträchtigt, der Polymer Gehalt im Film auch im unteren Bereich liegen wie z.B. im Bereich von 25 bis 50 Gew.-% oder bevorzugt im Bereich von 38 bis 40 Gew.-%.

Bei dem Wirkstoff kann es sich grundsätzlich um jeden oral verabreichbaren Wirkstoff handeln, wobei pharmazeutische Wirkstoffe bevorzugt sind. Pharmazeutische Wirkstoffe, die im Kontext der vorliegenden Erfindung für orale Applikationen geeignet sind, sind beispielsweise antiallergische Mittel, antiarrhythmische Mittel, Antibiotika, antidiabetische Mittel, antiepileptische Mittel, Antihistaminika, Antitussiva, cardiotronische Mittel, Diuretika, blutdrucksenkende Mittel, Betäubungsmittel, Nervenmuskelblocker, Sexualhormone und Vasopressoren. Spezifische Beispiele sind Acetaminophen, Adrenalin, Alprazolam, Amlodipin, Anastrozol, Apomorphin, Aripiprazol, Atorvastatin, Baclofen, Benzocain, Benzocain/Menthol, Benzydamin, Buprenorphin, Buprenorphin/Naloxon, Buprenorphin/Naloxon/Cetirizin, Cetirizin, Chlorpheniramin, Clomipramin, Dexamethason, Dextromethorphan, Dextromethorphan/Phenylephrin, Diclofenac, Diphenhydramin, Diphenhydramin/Phenylephrin, Donepezil Dronabinol, Epinephrin, Escitalopram, Famotidin, Fentanyl, Glimepirid, GLP-1 Peptiden, Granisetron, Insulin, Insulin Nanopartikel, Insulin/GLP-1 Nanopartikel, Ketamin, Ketoprofen, Ketotifen, Koffein, Levocetirizin, Loperamid, Loratadin, Meclizin, Methylphenidat, Midazolam, Mirodenafil, Montelukast, Multimeric-001, Naloxon, Nikotin, Nitroglycerin, Olanzapin, Olopatadin, Ondansetron, Oxybutynin, Pektin, Pektin/Menthol, Pectin/Ascorbinsäure, PediaSUNAT (Artesunat und Amodiaquin), Piroxicam, Phenylephrin, Prednisolon, Pseudoephedrin, Risperidon, Rivastigmin, Rizatriptan, Selegiline, Senna Glykosiden, Sildenafil Zitrat, Simethicon, Sumatriptan, Tadalafil, Testosteron, Triamcinolon Azetonid, Triptan, Tropicamide, Voglibose, Zolmitriptan, Zolpidem, oder pharmazeutisch akzeptable Salze dieser Verbindungen. Der pharmazeutische Wirkstoff kann auch als Mischung von unterschiedlichen Wirkstoffen vorliegen. Als nicht pharmazeutische Wirkstoffe kann die erfindungsgemäße Verabreichungsform z.B. Wirkstoffe für die Mundhygiene, wie Menthol, enthalten.

Der mindestens eine pharmazeutische Wirkstoff ist vorzugsweise in einer konstanten Konzentration in dem Film enthalten und weist insofern eine Gleichförmigkeit des Wirkstoffgehalts ("content uniformity") von 85 bis 115% des Sollgehalts auf, vorzugsweise 90 bis 110% des Sollgehalts und besonders bevorzugt 95 bis 105% des Sollgehalts auf, bezogen auf die Fläche einer Einheitsdosis des Films (vor dem Aufrollen).

Der Wirkstoffgehalt pro Dosiereinheit eines nach dem erfindungsgemäßen Verfahren hergestellten aufgerollten Films beträgt bis zu 500 mg, vorzugsweise bis 250 mg, besonders bevorzugt bis 230 mg, weiter bevorzugt bis 200 mg und noch weiter bevorzugt bis 100 mg. Auf der anderen Seite kann der Mindestwirkstoffgehalt pro Dosiereinheit vorzugsweise 5 mg, weiter bevorzugt 10 mg und meist bevorzugt 12 mg betragen. Je nach Anwendungsfall kann die Wirkstoffmenge auch im oberen Bereich der vorstehenden Angaben liegen, beispielsweise im Bereich von mehr als 50 bis 100 mg oder 30 bis 50 mg.

Die auf die Fläche der erfindungsgemäßen Verabreichungsform vor dem Aufrollen relativierte Wirkstoffmenge liegt zweckmäßig im Bereich von 1 bis 15 mg/cm² und bevorzugt 2,8 bis 10 mg/cm².

Der erfindungsgemäße als Filmverabreichungsform geeignete Film kann neben dem bereits erwähnten filmbildenden Polymer weitere Inhaltsstoffe enthalten, insbesondere Hilfsstoffe, die ausgewählt sind aus der Gruppe, umfassend Farbstoffe, Aromastoffe, insbesondere Geschmacks- und/oder Geruchsstoffe, Süßstoffe, geschmacksmaskierende Mittel, Tenside, Enhancer, pH-Regulatoren, Konservierungsmittel und/oder Antioxidationsmittel. Besonders vorteilhaft ist der Zusatz von Geschmacks-, Geruchs- und Aromastoffen, einzeln oder in Kombination. Ein geeignetes geschmacksmaskierendes Mittel ist beispielsweise ein Ionentauscherharz. Bevorzugt enthält der als Filmverabreichungsform geeignete Film Geschmacks-, Geruchs- und Aromastoffen, einzeln oder in Kombination.

Darüber hinaus kann der als orale Filmverabreichungsform geeignete Film ein Pigment oder ein UV-absorbierendes Mittel enthalten, das einen lichtempfindlichen in den Film eingebrachten Wirkstoff vor UV-Licht schützt. Neben den im Vorstehenden erwähnten Hilfsstoffen kann der als orale Filmverabreichungsform geeignete Film auch zusätzlich Bestandteile zur Optimierung seiner Flexibilitäts- und/oder physikalischen Eigenschaften enthalten, wie beispielsweise einen Weichmacher und/oder ein Feuchthaltemittel. Bevorzugte Weichmacher und/oder Feuchthaltemittel sind im Kontext der vorliegenden Erfindung zum Beispiel ausgewählt aus der Gruppe, umfassend Glycerin, Propylenglykol, Polyethylenglykol sowie Zitronensäureester. Ganz besonders bevorzugt als Weichmacher ist Glycerin.

Der als Filmverabreichungsform geeignete Film ist als verfestigter Schaum ausgeführt. Es kann ein eingebrachtes Gas, wie zum Beispiel Luft, Stickstoff oder CO₂, oder ein anderes Gas enthalten sein.

Geeignete Grundmaterialien und Verfahren zur Herstellung von entsprechenden verfestigten Schaumformulierungen sind beispielsweise in der EP 1 296 661 A2, der EP 1 959 921 A2 oder der WO 2018/224591 A1 beschrieben.

Die Hohlräume des Schaums können jeweils isoliert voneinander in der Polymermatrix vorliegen, vorzugsweise in Form von verfestigten Blasen.

Gemäß einer anderen Ausführungsform ist vorgesehen, dass die Hohlräume miteinander in Verbindung stehen, vorzugsweise indem sie ein zusammenhängendes, die Matrix durchdringendes Kanalsystem bilden.

Die Hohlräume des Schaums haben zweckmäßig einen Volumenanteil von 5 bis 98 %, bevorzugt von 30 bis 80 %, bezogen auf das Gesamtvolumen des Films (d.h. für den Volumenanteil sind gegebenenfalls vorhandene selbstklebende Schichten nicht zu berücksichtigen). Auf diese Weise wird eine beschleunigte Lösung der Filmverabreichungsform in günstiger Weise beeinflusst.

Ein weiterer wichtiger Parameter, der die Eigenschaften der erfindungsgemäßen Filmverabreichungsform auf Basis eines Schaums beeinflusst, ist der Durchmesser der Hohlräume oder Blasen. Die Blasen oder Hohlräume werden vorzugsweise mit Hilfe einer Schaumaufschlagmaschine erzeugt, mit der der Durchmesser der Blasen in einem weiten Bereich, fast beliebig, eingestellt werden kann. So kann der Durchmesser der Blasen oder Hohlräume im Bereich von 0,01 bis 60 µm liegen. Besonders bevorzugt liegt der Durchmesser im Bereich von 10 und 50 µm. Die Dicke der Filmschicht des als orale Filmverabreichungsform geeigneten Films liegt vorzugsweise im Bereich von etwa 0,01 bis etwa 2 mm und besonders bevorzugt im Bereich von 0,02 bis etwa 1 mm.

Auch in Bezug auf das Material, aus dem die selbstklebende Schicht gebildet ist, unterliegt die vorliegende Erfindung keinerlei relevanten Beschränkungen, mit der Maßgabe, dass die selbstklebende Schicht auf einem pharmazeutisch akzeptablen wasserlöslichen oder wasserdispergierbaren Polymer beruhen sollte. Ein geeignetes wasserlösliches oder wasserdispergierbares Polymer ist zum Beispiel Plastoid E35H (weichgestelltes Eudragit E100; als Modifikatoren werden Laurinsäure, Adipinsäure und Glycerin zugesetzt). Weitere geeignete wasserlösliche oder -dispergierbare Polymere sind beispielsweise Schellack, ein Vinylpyrrolidon-/Vinylacetat-Copolymer, ein Polyvinylcaprolactam-/Polyvinylacetat-/Polyethylenglykol-Copolymer, Hydroxypropylcellulose oder Hydroxypropylmethylcellulose und/oder Polyvinylpyrrolidon. Ein im Rahmen der vorliegen Erfindung ganz besonders bevorzugtes wasserlösliches Polymer ist Polyvinylpyrrolidon.

Die vorgenannten wasserlöslichen Polymere werden zweckmäßig mit einem Weichmacher kombiniert. Als geeignete Weichmacher sind beispielsweise Glycerin, Polyethylenglykol, insbesondere Polyethylenglykol 200, Sorbitol und/oder Tributylcitrat zu nennen, von denen Glycerin, Polyethylenglykol 200 und/oder Triutylcitrat als besonders geeignet angegeben werden können. Ein ganz besonders geeigneter Weichmacher ist Glycerin.

In Bezug auf das Verhältnis von wasserlöslichem Polymer zu Weichmacher unterliegt der Klebstoff keinen relevanten Beschränkungen, solange das Verhältnis so eingestellt ist, dass die Mischung ausreichend klebrig und verarbeitbar ist. Als günstiges Mischungsverhältnis kann ein Verhältnis von wasserlöslichem Polymer zu Weichmacher von etwa 85 bis 50 zu etwa 15 bis 50, bevorzugt von etwa 85 bis 65 zu etwa 15 zu 35, noch bevorzugter von etwa 80 bis 60 zu etwa 20 bis 40, noch weiter bevorzugt von etwa 80 bis 50 zu etwa 20 bis 50, noch weiter bevorzugt von etwa 82 bis 68 zu etwa 18 zu 32 und am meisten bevorzugt von etwa 80 bis 70 zu etwa 20 bis 30 angegeben werden.

Als geeigneter Anteil des wasserlöslichen oder -dispergierbaren Polymers in der selbstklebenden Schicht kann ein Anteil von etwa 50 bis 90 und bevorzugt von etwa 60 bis 85% an dem Film angegeben werden.

Bei dieser Art der Aktivierung der (ersten) Oberfläche des in Schritt (a) hergestellten Films bleibt nach dem Aufrollen eine - wenn auch nur vergleichsweise extrem dünne - Zwischenschicht zwischen der aktivierten (ersten) Oberfläche und der nicht-aktivierten (zweiten) Oberfläche des Films im aufgerollten Laminat bestehen, d.h. die Sichtdicke der selbstklebenden Schicht ist vorzugsweise um mindestens den Faktor 2, weiter bevorzugt um mindestens den Faktor 4 und noch weiter bevorzugt um mindestens den Faktor 8 geringer als die Schichtdicke des Wirkstoff-haltigen Films der Filmverabreichungsform.

Das Aufrollen des aktivierten Films kann zweckmäßig mittels rotierender Bürsten im Winkel von etwa 45° erfolgen. Es ist zudem möglich, einen Wickelkern auf das flächenförmig vorliegende Laminat aufzubringen und das Laminat anschließend über diesen Wickelkern aufzuwickeln. Nach Beendigung kann der Wickelkern entfernt werden, so dass eine zentrale Aussparung entsteht. Diese kann einen Durchmesser von etwa 0,5 bis 30 mm aufweisen, bevorzugt von etwa 1 bis 10 mm und besonders bevorzugt von etwa 2 bis 5 mm.

Alternativ ist es möglich, dass der Wickelkern als Bestandteil der Zubereitung im System verbleibt, wobei dieser Wickelkern kompakt oder hohl, als Ring vorliegend ausgeprägt sein, einen Wirkstoff enthaltend oder weitgehend wirkstofffrei ausgeführt sein kann. Darüber hinaus kann die Breite des Wickelkerns die maximale Breite des Laminats überschreiten. Der Durchmesser des Wickelkerns liegt zweckmäßig bei etwa 0,5 bis 30 mm, bevorzugt bei etwa 1 bis 10 mm, und besonders bevorzugt bei etwa 2 bis 5 mm.

Das aufgerollte Laminat kann nach dem Aufrollen in Schritt (c) in kleinere einzelne Portionen unterteilt werden. Zu diesem Zweck enthält das erfindungsgemäße Verfahren zweckmäßig einen Schritt des Zerteilens des in (c) hergestellten aufgerollten Laminats. Dadurch entstehen Abschnitte derartiger Laminatrollen mit konstanter Breite, z.B. von 1 bis 4 cm und insbesondere 1,5 bis 2,5 cm.

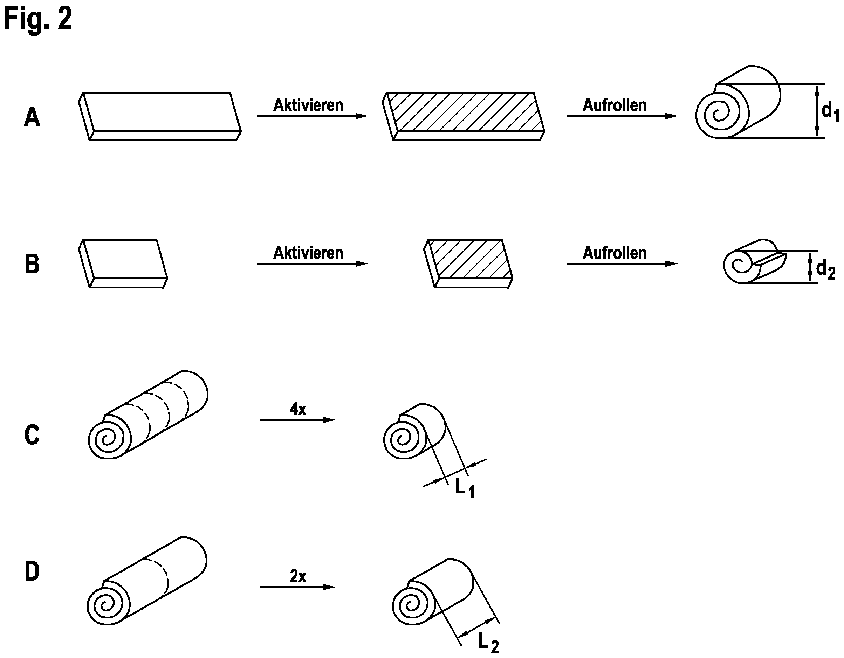

Die Flexibilität bei der Herstellung unterschiedlicher Formen und Größen der erfindungsgemäßen aufgerollten Laminate ist in Fig. 2 illustriert. In Fig. 2 A wird ein vergleichsweise langer Film in einem ersten Schritt auf einer Oberfläche aktiviert und anschließend aufgerollt, wobei ein vergleichsweise dickes aufgerolltes Laminat erhalten wird (d1). In Fig. 2 B wird ein kürzerer Film auf einer Oberfläche aktiviert und anschließend aufgerollt, wobei ein dünneres aufgerolltes Laminat erhalten wird (d2 < d1).

In Fig. 2C und 2D wird die Flexibilität anhand der einstellbaren Länge der aufgerollten Laminate illustriert, mit der sich z.B. die gewünschte Wirkstoffmenge auf Basis eines Wirkstofffilms mit durch den Hersteller vorbestimmter Wirkstoffkonzentration an die Bedürfnisse des einzelnen Patienten anpassen lässt. Ein voraufgerolltes Filmlaminat kann z.B. in 4 gleiche Teile zerschnitten (C) oder in 2 gleiche Teile (D) zerschnitten werden, wobei Filmlaminatstücke unterschiedlicher Länge (L₂ = 2 L₁) erhalten werden.

Gemäß einem zweiten Aspekt betrifft die vorliegende Erfindung ein aufgerolltes Laminat, das nach dem im Vorstehenden beschriebenen Verfahren herstellbar oder hergestellt ist. Das aufgerollte Laminat weist vorzugsweise ein Gewicht von etwa 100 bis 1000 mg und bevorzugt etwa 200 bis 800 mg auf.

Weiterhin bevorzugt ist für das erfindungsgemäße aufgerollte Laminat ein Restlösemittel- bzw. Restwassergehalt von weniger als 10 Gew.-% und insbesondere weniger als 5 Gew.-%.

Für das erfindungsgemäße aufgerollte Laminat ist es wesentlich, dass es im Wesentlichen (d.h. zu mehr als 95 Gew.-% und bevorzugt zu mehr als 98 Gew.-%) und bevorzugt vollständig aus wasserlöslichen oder wasserdispergierbaren Bestandteilen besteht, so dass sich das Laminat bei Kontakt mit Wasser relativ schnell auflöst oder zerfällt und den darin einbezogenen Wirkstoff freisetzt. Zweckmäßig weisen hierzu sowohl die wirkstoffhaltige Schicht als auch eine eventuell vorhandene selbstklebende Schicht eine Auflösungsgeschwindigkeit (bestimmt für den einzelnen Film gemäß angepasster Prüfung A für die Zerfallszeit von Kapseln und Tabletten, europäisches Arzneibuch (2011), wobei die jeweilige Schicht direkt am Schaft des Zerfallstesters befestigt wird) von weniger als etwa 5 min, bevorzugt weniger als etwa 120 s und weiter bevorzugt weniger als etwa 50 s auf. Falls das erfindungsgemäße aufgerollte Laminat einen Ionentauscher als geschmacksmaskierendes Mittel enthält, gilt dieser im Kontext dieser Erfindung als wasserlöslicher oder wasserdispergierbarer Bestandteil.

Das erfindungsgemäße aufgerollte Laminat eignet sich insbesondere für eine orale Verabreichung, einschließlich einer buccalen, gingivalen oder sublingualen Verabreichung, von Wirkstoffen oder einer Verabreichung am Gaumen.

Das aufgerollte Laminat kann in vielfältiger Weise modifiziert werden. So kann es zum Beispiel in eine Kapsel gefüllt werden, die dann geschluckt werden kann.

Alternativ kann das aufgerollte Laminat mit einem Magensaft-löslichen Überzug zum Auflösen im Magen oder mit Magensaft-resistenten Überzug zum Auflösen im Darm modifiziert werden. Durch die aufgerollte Form ist eine Applikation in alle Körperöffnungen (zum Beispiel im Mund, in Nasenlöcher, Gehörgänge, After, Vagina) problemlos möglich. Zudem kann das aufgerollte Material in einem Spender nach Bedarf gespendet und geschnitten werden. Durch die Rollenform lässt sich das Material auch gut in der Wangentasche oder unter der Zunge applizieren ohne zu stören. Durch das Auflösen kann ein sehr hohes Flächengewicht von > 100 mg/cm (bei einem Durchmesser von beispielsweise 0,5 mm) erreicht werden. Es lassen sich zudem relativ lange Stücke applizieren (< 3 cm, d.h., mehr als 300 mg).

Im Folgenden soll die vorliegende Erfindung anhand eines Ausführungsbeispiels näher illustriert werden, das jedoch nicht als in irgendeiner Weise beschränkend für den Schutzumfang der Anmeldung angesehen werden sollte.

### Beispiel 1

Es wurde ein geschäumter Film mit der in der nachfolgenden Tabelle für die Wirkstoffschicht angegebenen Zusammensetzung (Schichtdicke: ~ 0,4 mm; Flächengewicht 200 g/m²) hergestellt.

| Material | Funktion | Anteil (% trocken) |
|---|---|---|
| **Klebeschicht** | | |
| PVP VA64 | Polymer | 80 |
| Glycerin | Weichmacher | 20 |

| **Wirkstoffschicht** | | |
|---|---|---|
| Polyvinylalkohol¹ | Polymer | 77,6 |
| FD&C Red 40 | Farbstoff | 0,4 |
| Saccharin Na | Süßstoff | 2,0 |
| Sucralose | Süßstoff | 4,0 |
| Glycerin | Weichmacher/Feuchthaltemittel | 9,0 |
| Cherry Flavour | Aroma | 6,0 |
| Menthol | Aroma | 1,0 |

| | | |
|---|---|---|
| ¹ = 35 % in Wasser | | |

Für die Herstellung wurden die weiteren Inhaltsstoffe in eine PVA-Vorlösung eingerührt und unter weiterem Rühren mit Wasser auf einen Feststoffgehalt von 34,5 % eingestellt. Die so erhaltene Mischung wurde anschließend 2h lang homogenisiert, mit einer Schaummaschine aufgeschäumt und mit einem Roll Coater zu einem Schaumfilm verarbeitet. Der Restlösemittelgehalt des getrockneten Films betrug etwa 3%.

Auf diesen Film wurde die Klebeschicht aus PVP VA64 und Glycerin durch Transfer von einem dehäsiv ausgerüsteten Träger appliziert. Das so hergestellte Laminat wurde mit innenliegender Klebeschicht aufgerollt und in Portionsstücke geschnitten.

## Patentansprüche

1. Verfahren zur Herstellung einer wasserlöslichen oder wasserdispergierbaren Wirkstoff-Verabreichungsform umfassend
(a) Herstellen eines als orale Filmverabreichungsform geeigneten Films mit einem Gehalt an Wirkstoff, wobei die Filmverabreichungsform als verfestigter Schaum vorliegt,
(b) Aktivieren einer Oberfläche des in (a) hergestellten Films zur Generierung einer klebrigen Oberfläche und
(c) Aufrollen des in (b) aktivierten Films unter Bildung eines aufgerollten Laminats.

2. Verfahren gemäß Anspruch 1, **dadurch gekennzeichnet, dass** das Aktivieren der Oberfläche und das Generieren einer klebrigen Oberfläche durch Quellen, Anlösen oder Anschmelzen der Oberfläche erfolgt.

3. Verfahren gemäß Anspruch 1, **dadurch gekennzeichnet, dass** das Aktivieren der Oberfläche und das Generieren einer klebrigen Oberfläche durch das Aufbringen einer selbstklebenden Schicht erfolgt, wobei die selbstklebende Schicht aus einem wassererodierbaren Material und bevorzugt einem wasserlöslichen oder wasserdispergierbaren Material gebildet wird.

4. Verfahren gemäß mindestens einem der Ansprüche 1 bis 3, **dadurch gekennzeichnet, dass** der als orale Filmverabreichungsform geeignete Film auf einem filmbildenden Polymer ausgewählt aus Polyvinylalkohol, Polyethylenglycol, Polyethylenoxid, Cellulosederivate, Pullulan, Gelatine und Agar, bevorzugt auf Polyvinylalkohol, beruht.

5. Verfahren gemäß Anspruch 4, **dadurch gekennzeichnet, dass** das filmbildende Polymer einen Anteil von 25 bis 85 und bevorzugt von 60 bis 80 Gew.-% an dem Film ausmacht.

6. Verfahren gemäß mindestens einem der vorstehenden Ansprüche, **dadurch gekennzeichnet, dass** der als orale Filmverabreichungsform geeignete Film mindestens einen Hilfsstoff ausgewählt aus Geschmacks- oder Aromastoffen, Farbstoffen, Weichmachern und Süßstoffen enthält.

7. Verfahren gemäß mindestens einem der vorstehenden Ansprüche, **dadurch gekennzeichnet, dass** der Wirkstoff ein pharmazeutischer Wirkstoff, ausgewählt aus der Gruppe umfassend Acetaminophen, Adrenalin, Alprazolam, Amlodipin, Anastrozol, Apomorphin, Aripiprazol, Atorvastatin, Baclofen, Benzocain, Benzocain/Menthol, Benzydamin, Buprenorphin, Buprenorphin/Naloxon, Buprenorphin/Naloxon/Cetirizin, Cetirizin, Chlorpheniramin, Clomipramin, Dexamethason, Dextromethorphan, Dextromethorphan/Phenylephrin, Diclofenac, Diphenhydramin, Diphenhydramin/Phenylephrin, Donepezil Dronabinol, Epinephrin, Escitalopram, Famotidin, Fentanyl, Glimepirid, GLP-1 Peptiden, Granisetron, Insulin, Insulin Nanopartikel, Insulin/GLP-1 Nanopartikel, Ketamin, Ketoprofen, Ketotifen, Koffein, Levocetirizin, Loperamid, Loratadin, Meclizin, Methylphenidat, Midazolam, Mirodenafil, Montelukast, Multimeric-001, Naloxon, Nikotin, Nitroglycerin, Olanzapin, Olopatadin, Ondansetron, Oxybutynin, Pektin, Pektin/Menthol, Pectin/Ascorbinsäure, PediaSUNAT (Artesunat und Amodiaquin), Piroxicam, Phenylephrin, Prednisolon, Pseudoephedrin, Risperidon, Rivastigmin, Rizatriptan, Selegiline, Senna Glykosiden, Sildenafil Zitrat, Simethicon, Sumatriptan, Tadalafil, Testosteron, Triamcinolon Azetonid, Triptan, Tropicamide, Voglibose, Zolmitriptan, Zolpidem, oder pharmazeutisch akzeptablen Salzen dieser Verbindungen, ist.

8. Verfahren gemäß Anspruch 3 oder einem davon abhängigen Anspruch, **dadurch gekennzeichnet, dass** die selbstklebende Schicht auf einem wasserlöslichen oder wasserdispergierbaren Polymer, bevorzugt auf Polyvinylpyrrolidon, beruht.

9. Verfahren gemäß Anspruch 8, **dadurch gekennzeichnet, dass** das Wasser-lösliche oder -dispergierbare Polymer einen Anteil von 50 bis 90 und bevorzugt von 60 bis 85 Gew.-% an der selbstklebenden Schicht ausmacht.

10. Verfahren gemäß mindestens einem der vorstehenden Ansprüche, **dadurch gekennzeichnet, dass** der als orale Filmverabreichungsform geeignete Film oder der selbstklebende Film einen Weichmacher und/oder ein Feuchthaltemittel, bevorzugt in Form von Glycerin, enthält.

11. Aufgerolltes Laminat, herstellbar nach einem Verfahren gemäß einem der Ansprüche 1 bis 10.

12. Aufgerolltes Laminat nach Anspruch 11, **dadurch gekennzeichnet, dass** es ein Gewicht von 100 bis 1000 mg, und bevorzugt von 200 bis 800 mg aufweist.

## Claims

1. A method for producing a water-soluble or water-dispersible active-ingredient-administering form comprising
(a) producing a film, suitable as oral film-administering form, having a content of active ingredient, wherein the film-administering form is present as a solidified foam,
(b) activating a surface of the film produced in (a) in order to generate a tacky surface, and
(c) rolling up the film activated in (b) to form a rolled-up laminate.

2. The method according to claim 1, **characterised in that** the surface is activated and a tacky surface is generated by swelling, etching or melting of the surface.

3. The method according to claim 1, **characterised in that** the surface is activated and a tacky surface is generated by applying a self-adhering layer, wherein the self-adhering layer is formed from a water-erodible material and preferably a water-soluble or water-dispersible material.

4. The method according to at least one of claims 1 to 3, **characterised in that** the film suitable as an oral film-administering film is based on a film-forming polymer, selected from polyvinyl alcohol, polyethylene glycol, polyethylene oxide, cellulose derivatives, pullulan, gelatines, and agar, preferably on polyvinyl alcohol.

5. The method according to claim 4, **characterised in that** the film-forming polymer accounts for a proportion of from 25 to 85 and preferably from 60 to 80 wt.% in the film.

6. The method according to at least one of the preceding claims, **characterised in that** the film suitable as oral film-administering form contains at least one excipient selected from flavourings or aromatic substances, colouring agents, plasticisers and sweeteners.

7. The method according to at least one of the preceding claims, **characterised in that** the active ingredient is an active pharmaceutical ingredient selected from the group comprising acetaminophen, adrenaline, alprazolam, amlodipine, anastrozole, apomorphine, aripiprazole, atorvastatin, baclofen, benzocaine, benzocaine/menthol, benzydamine, buprenorphine, buprenorphine/naloxone, buprenorphine/naloxone/cetirizine, cetirizine, chlorpheniramine, clomipramine, dexamethasone, dextromethorphan, dextromethorphan/phenylephrine, diclofenac, diphenhydramine, diphenhydramine/phenylephrine, donepezil, dronabinol, epinephrine, escitalopram, famotidine, fentanyl, glimepiride, GLP-1 peptides, granisetron, insulin, insulin nanoparticles, insulin/GLP-1 nanoparticles, ketamine, ketoprofen, ketotifen, caffeine, levocetirizine, loperamide, loratadine, meclizine, methylphenidate, midazolam, mirodenafil, montelukast, multimeric-001, naloxone, nicotine, nitroglycerin, olanzapine, olopatadine, ondansetron, oxybutynin, pectin, pectin/menthol, pectin/ascorbic acid, PediaSUNAT (artesunate and amodiaquine), piroxicam, phenylephrine, prednisolone, pseudoephedrine, risperidone, rivastigmine, rizatriptan, selegiline, Senna glycosides, sildenafil citrate, simethicone, sumatriptan, tadalafil, testosterone, triamcinolone acetonide, triptan, tropicamide, voglibose, zolmitriptan, zolpidem, or pharmaceutically acceptable salts of these compounds.

8. The method according to claim 3 or a claim dependent thereon, **characterised in that** the self-adhering layer is based on a water-soluble or water-dispersible polymer, preferably on polyvinylpyrrolidone.

9. The method according to claim 8, **characterised in that** the water-soluble or water-dispersible polymer accounts for a proportion of from 50 to 90 and preferably from 60 to 85 wt.% in the self-adhering layer.

10. The method according to at least one of the preceding claims, **characterised in that** the film suitable as oral film-administering form or the self-adhering film contains a plasticiser and/or a humectant, preferably in the form of glycerol.

11. A rolled-up laminate, producible by a method according to any one of claims 1 to 10.

12. The rolled-up laminate according to claim 11, **characterised in that** it has a weight of from 100 to 1000 mg, and preferably from 200 to 800 mg.

## Revendications

1. Procédé de préparation d'une forme d'administration de principe actif soluble ou dispersible dans l'eau comprenant
(a) la préparation d'un film adapté en tant que forme d'administration de film par voie orale ayant une teneur en principe actif, la forme d'administration de film se présentant sous la forme d'une mousse solidifiée,
(b) l'activation d'une surface du film préparé au point (a) pour générer une surface collante et
(c) l'enroulement du film activé au point (b) pour former un stratifié enroulé.

2. Procédé selon la revendication 1, **caractérisé en ce que** l'activation de la surface et la génération d'une surface collante s'effectuent par gonflement, décollement ou fusion de la surface.

3. Procédé selon la revendication 1, **caractérisé en ce que** l'activation de la surface et la génération d'une surface collante s'effectuent par l'application d'une couche autoadhésive, la couche autoadhésive étant formée d'un matériau pouvant s'éroder à l'eau et de préférence d'un matériau soluble ou dispersible dans l'eau.

4. Procédé selon au moins l'une des revendications 1 à 3, **caractérisé en ce que** le film adapté en tant que forme d'administration de film par voie orale est à base d'un polymère filmogène choisi parmi l'alcool polyvinylique, le polyéthylène glycol, l'oxyde de polyéthylène, les dérivés de cellulose, le pullulane, la gélatine et l'agar, de préférence à base d'alcool polyvinylique.

5. Procédé selon la revendication 4, **caractérisé en ce que** le polymère filmogène représente une proportion de 25 à 85 et de préférence de 60 à 80 % en poids du film.

6. Procédé selon au moins l'une des revendications précédentes, **caractérisé en ce que** le film adapté en tant que forme d'administration de film par voie orale contient au moins un excipient choisi parmi les arômes, les colorants, les plastifiants et les édulcorants.

7. Procédé selon au moins l'une des revendications précédentes, **caractérisé en ce que** le principe actif est un principe actif pharmaceutique choisi dans le groupe comprenant le paracétamol, l'adrénaline, l'alprazolam, l'amlodipine, l'anastrozole, l'apomorphine, l'aripiprazole, l'atorvastatine, le baclofène, la benzocaïne, le benzocaïne/menthol, la benzydamine, la buprénorphine, la buprénorphine/naloxone, la buprénorphine/naloxone/cétirizine, la cétirizine, la chlorphéniramine, la clomipramine, la dexaméthasone, le dextrométhorphane, la dextrométhorphane/phényléphrine, le diclofénac, la diphénhydramine, la diphénhydramine/phényléphrine, le donépezil dronabinol, l'épinéphrine, l'escitalopram, la famotidine, le fentanyl, le glimépiride, les peptides GLP-1, le granisétron, l'insuline, les nanoparticules d'insuline, les nanoparticules d'insuline/GLP-1, la kétamine, le kétoprofène, le kétotifène, la caféine, la lévocétirizine, le lopéramide, la loratadine, la méclozine, le méthylphénidate, le midazolam, le mirodénafil, le montelukast, le multimère-001, la naloxone, la nicotine, la nitroglycérine, l'olanzapine, l'olopatadine, l'ondansétron, l'oxybutynine, la pectine, le pectine/menthol, le pectine/acide ascorbique, le PediaSUNAT (artésunate et amodiaquine), le piroxicam, la phényléphrine, la prednisolone, la pseudoéphédrine, la rispéridone, la rivastigmine, le rizatriptan, la sélégiline, les glycosides de séné, le citrate de sildénafil, la siméthicone, le sumatriptan, le tadalafil, la testostérone, l'acétonide de triamcinolone, le triptan, le tropicamide, le voglibose, le zolmitriptan, le zolpidem, ou des sels pharmaceutiquement acceptables de ces composés.

8. Procédé selon la revendication 3 ou une revendication dépendante de celle-ci, **caractérisé en ce que** la couche autoadhésive est à base d'un polymère soluble ou dispersible dans l'eau, de préférence à base de polyvinylpyrrolidone.

9. Procédé selon la revendication 8, **caractérisé en ce que** le polymère soluble ou dispersible dans l'eau représente une proportion de 50 à 90 et de préférence de 60 à 85 % en poids de la couche autoadhésive.

10. Procédé selon au moins l'une des revendications précédentes, **caractérisé en ce que** le film adapté en tant que forme d'administration de film par voie orale ou le film autoadhésif contient un plastifiant et/ou un agent humectant, de préférence sous forme de glycérine.

11. Stratifié enroulé, pouvant être préparé d'après un procédé selon l'une des revendications 1 à 10.

12. Stratifié enroulé selon la revendication 11, **caractérisé en ce qu'**il présente un poids de 100 à 1000 mg, et de préférence de 200 à 800 mg.
